# EUROPÄISCHE PATENTSCHRIFT

(11) **EP 2 755 964 B1**
(45) Veröffentlichungstag und Bekanntmachung des Hinweises auf die Patenterteilung: **22.06.2016**
(21) Anmeldenummer: 12761936.9
(22) Anmeldetag: 12.09.2012
(51) Int. Cl.: C07D 401/12, A61K 31/4439, A61P 31/12

(54) **SULFONSÄURESALZE HETEROCYCLYLAMID-SUBSTITUIERTER IMIDAZOLE**
SULFONIC ACID SALTS OF HETEROCYCLYLAMIDE-SUBSTITUTED IMIDAZOLES
SELS D'ACIDE SULFONIQUE D'IMIDAZOLES SUBSTITUÉS PAR HÉTÉROCYCLYLAMIDE

(30) Priorität: 14.09.2011 DE 102011113749
(43) Veröffentlichungstag der Anmeldung: 23.07.2014
(73) Patentinhaber: AiCuris Anti-infective Cures GmbH, 42117 Wuppertal (DE)
(72) Erfinder: SCHWAB, Wilfried, 42551 Velbert (DE); SCHIFFER, Guido, 42111 Wuppertal (DE); VOEGTLI, Kurt, CH-5273 Oberhofen (CH); KYAS, Andreas, 79664 Wehr (DE); OSSWALD, Gerd, CH-5032 Aarau Rohr (CH)
(74) Vertreter: Kilger, Ute
(86) Internationale Anmeldenummer: PCT/EP2012/067814
(87) Internationale Veröffentlichungsnummer: WO 2013/037812

(56) Entgegenhaltungen:
- WO-A2-2006/089664
- BERGE ET AL.: "Pharmaceutical salts", J. PHARM. SCI., [Online] Bd. 66, Nr. 1, 1. Januar 1977 (1977-01-01) , Seiten 1-19, XP002675560, ISSN: 0022-3549, DOI: 10.1002/JPS.2600660104
- Stahl et al. (eds.): "Handbook of pharmaceutical salts: properties, selection, and use", 1. Januar 2002 (2002-01-01), Wiley VCH, Weinheim, XP003024996, ISBN: 978-3-906390-26-0 Seiten 212-217, Seite 213, Absätze 8.1.5 und 8.1.7.

## Beschreibung

Die vorliegende Erfindung betrifft Methansulfonsäuresalze von Verbindungen der Formel 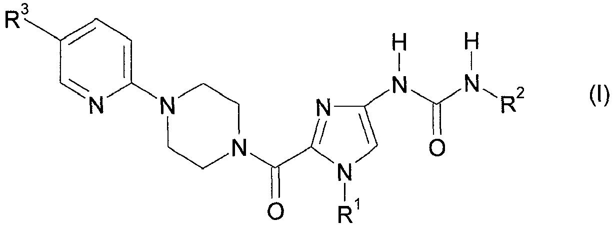 in welcher
- R¹: für Methyl, Ethyl, Butyl oder Cyclopropylmethyl steht,
- R²: für Phenyl steht, wobei Phenyl substituiert ist mit einem Substituenten ausgewählt aus der Gruppe bestehend aus Trifluormethoxy und Difluormethoxy, und
- R³: für Wasserstoff, Methyl, Chlor, Methoxy oder Trifluormethyl steht.

Die Erfindung betrifft ferner Verfahren zu ihrer Herstellung, entsprechende Arzneimittel und die genannten Methansulfonsäuresalze und Arzneimittel zur Behandlung und/oder Prophylaxe von Krankheiten, insbesondere zur Verwendung als antivirale Mittel, insbesondere gegen Cytomegalieviren.

Die Verbindungen der Formel (I) sind z.B. aus der WO 2006/089664 bekannt und wurden von der Anmelderin als aussichtsreiche Kandidaten für antiviral wirksame Substanzen insbesondere zur Bekämpfung von Infektionen mit dem humanen Cytomegalievirus (HCMV) entwickelt. Bei der Entwicklung hat sich allerdings herausgestellt, dass die Substanzen in wässrigen, sowie stark polaren Lösungsmitteln eine ungenügende Löslichkeit zeigten. Die Probleme im Hinblick auf die Löslichkeit wurden ferner noch dadurch verschärft, dass die Verbindungen auch unter den im menschlichen Magen vorliegenden Bedingungen (ca. 0.1M HCl, pH∼1), bei denen von der *in situ* Bildung eines HCl-Salzes ausgegangen werden kann, eine nicht ausreichende Löslichkeit zeigten.

Es ist somit eine Aufgabe der Erfindung Salze zu beschreiben, die im Vergleich zur freien Base der Verbindungen der Formel (I) eine deutlich verbesserte Löslichkeit zeigen. Ferner sollten diese Salze auch unter den üblichen Lagerbedingungen langzeitstabil sein. Insbesondere sollten die Verbindungen keine erhöhte Hygroskopie zeigen. Auch sollten die Salze in Gegenwart einer verdünnten HCl-Lösung sich nur langsam in das HCl-Salz umwandeln um auch unter Bedingungen, wie sie im menschlichen Magen vorliegen eine möglichst schnelle und gleichmäßige Freisetzung sicherzustellen.

Überraschenderweise wurde herausgefunden, dass die organischen Sulfonsäuresalze der Verbindungen der Formel (I) im Vergleich mit der freien Base, sowie einem breiten Spektrum anderer Salze der Verbindungen der Formel (I) eine überlegene Löslichkeit zeigen. Ferner zeigen diese Salze auch die für die Verwendung in Medikamenten nötige Langzeitstabilität. Es hat sich außerdem gezeigt, dass die erfindungsgemäßen Salze auch unter Bedingungen, die denen im menschlichen Magen entsprechen eine hohe und gleichmäßig Löslichkeit zeigen.

Gegenstand der Erfindung sind Salze der Verbindungen der Formel (I) mit Methansulfonsäure.

Salze organischer Sulfonsäuren sind im Rahmen der Offenbarung Addukte einer Reaktion einer Verbindung der Formel (I) mit einer organischen Sulfonsäure. Die Verbindungen der Formel (I) und die organischen Sulfonsäuren können hierbei in jedem Verhältnis vorliegen. Bevorzugt ist das Verhältnis dabei ganzzahlig (z.B. 1 : 1, 1 : 2, 1 : 3, 3:1; 2:1). Diese Salze können dabei durch eine direkte Umsetzung der Verbindungen der Formel (I) mit einer organischen Sulfonsäure oder durch Herstellen anderer Säuresalze der Verbindungen der Formel (I) gefolgt von einem Austausch des Gegenions hergestellt werden.

Als Solvate werden im Rahmen der Offenbarung solche Formen der offenbarungsgemäßen Verbindungen bezeichnet, welche durch Koordination mit Lösungsmittelmolekülen einen Komplex bilden. Hydrate sind eine spezielle Form der Solvate, bei denen die Koordination mit Wasser erfolgt.

Besonders bevorzugt im Rahmen der Erfindung sind die Dimesylatsalze.

Bevorzugt im Rahmen der Erfindung ist ein Salz mit der folgenden Formel:

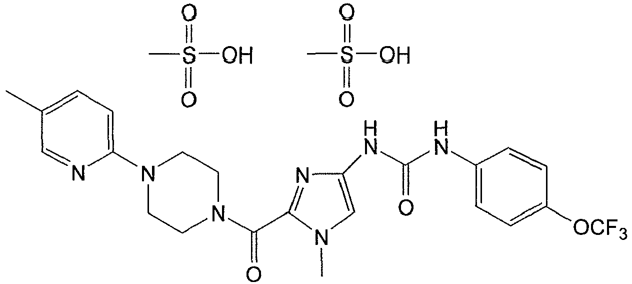

Bevorzugt im Rahmen der Erfindung ist insbesondere ein kristallines N-(1-Methyl-2-{[4-(5-methylpyridin-2-yl)piperazin-1-yl]-carbonyl}-1H-imidazol-4-yl)-N'-[4-trifluormethoxyphenyl]harnstoff Dimesylat, das im Pulver-XRD-Diffraktogramm charakteristische Peaks bei etwa 6.37, 11.77, 12.56, 17.17, 18.81, 20.34, 21.47, 23.04, 35.46 Grad 2Theta zeigt.

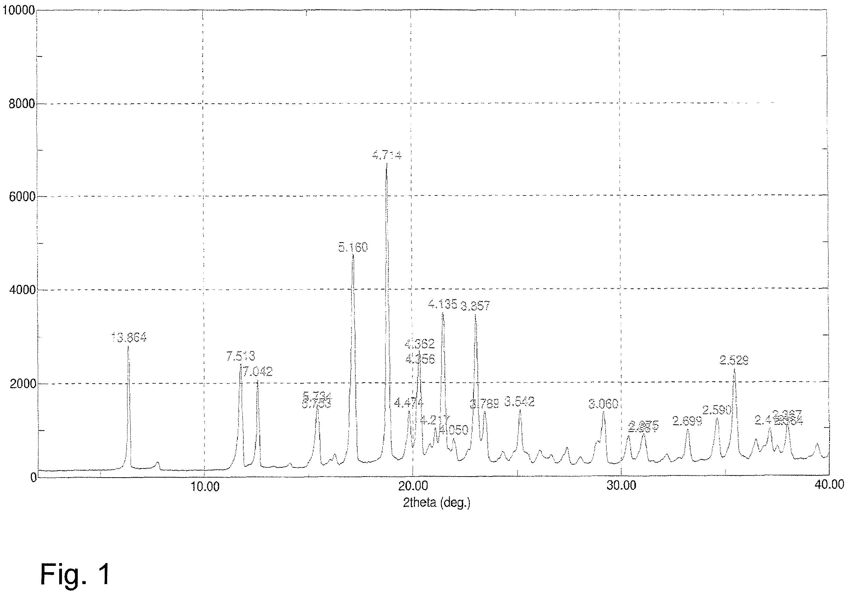

Bevorzugt im Rahmen der Erfindung ist ferner kristallines N-(1-Methyl-2-{[4-(5-methylpyridin-2-yl)piperazin-1-yl]-carbonyl}-1*H*-imidazol-4-yl)-N'-[4-trifluormethoxyphenyl]harnstoff Dimesylat, das ein Pulver-XRD-Diffraktogramm zeigt, wie in Fig. 1 dargestellt.

Die erfindungsgemäßen Salze werden im Allgemeinen durch Umsetzen einer Verbindung der Formel (I) mit Methansulfonsäure in einem Lösungsmittel hergestellt.

Es ist ferner möglich offenbarungsgemäße Salze durch Umsetzen eines Säuresalzes der Verbindungen der Formel (I), das kein Salz einer organischen Sulfonsäure ist, mit einer Quelle für Sulfonat-Anionen einer organischen Sulfonsäure in einem Lösungsmittel herzustellen.

Im letzteren Fall kann die Quelle für Sulfonat-Anionen eine organische Sulfonsäure oder ein Salz einer organischen Sulfonsäure sein.

Gegenstand der Offenbarung ist somit ferner ein Verfahren zur Herstellung von organischen Sulfonsäuresalzen der Verbindungen der Formel (I), das die Umsetzung von Verbindungen der Formel (I) oder Salzen von Verbindungen der Formel (I), die keine Säuresalze einer organischen Sulfonsäure sind, mit einer organischen Sulfonsäure oder einer Quelle für organische Sulfonat-Anionen in ein Lösungsmittel aufweist.

Das Lösungsmittel ist vorzugsweise so ausgewählt, dass es eine gute Balance zwischen der Löslichkeit der Verbindungen der Formel (I) beziehungsweise den Salzen der Verbindung der Formel (I), die keine Sulfonsäuresalze sind, und der organischen Sulfonsäure beziehungsweise der Quelle für Sulfonat-Anionen bietet. Vorzugsweise sollten die offenbarungsgemäßen Salze in dem verwendeten Lösungsmittel möglichst wenig löslich sein. Gegebenenfalls können die offenbarungsgemäßen Salze jedoch auch durch Zugabe eines Gegenlösungsmittels ausgefällt werden.

Beispiele für Lösungsmittel, die zur Herstellung der erfindungsgemäßen Salze verwendet werden, schließen Folgendes ein, nämlich Alkohole wie Methanol, Ethanol, n-Propanol, iso-Propanol und Butanol; Ether wie Diethylether, Methyl-*tert*.-butylether, 1,2-Dimethoxyethan, Dioxan oder Tetrahydrofuran; Kohlenwasserstoffe wie Benzol oder Toluol; oder andere Lösungsmittel wie Aceton, Essigester, Methylethylketon, Methylisobutylketon, Acetonitril, Heptan, Dimethylsulfoxid oder Dimethylformamid.

Gegebenenfalls wird zum Ausfällen der erfindungsgemäßen Salze ein Gegenlösungsmittel zugegeben. Beispiele für solchen Gegenlösungsmittel schließen Folgendes ein, nämlich Wasser und Alkohole wie Methanol, Ethanol oder Propanol.

Die so erhaltenen erfindungsgemäßen Salze können gegebenenfalls weiter bearbeitet, z.B. umkristallisiert oder mikronisiert werden, um deren physikalische Eigenschaften weiter auf den Verwendungszweck anzupassen.

Die zur Herstellung der erfindungsgemäßen Salze verwendeten Heterocyclylamid-substituierten Imidazole sind bekannt und können z.B. nach dem in der WO 2006/089664 beschriebene Verfahren hergestellt werden.

Insbesondere erfolgt die Herstellung der verwendeten Heterocyclylamidsubstituierten Imidazole durch Umsetzen von Verbindungen der Formel 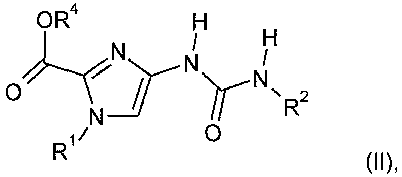 in welcher
- R¹ und R²: wie zuvor definiert sind, und
- R⁴: für Methyl oder Ethyl steht,
in der ersten Stufe mit einer Base und in der zweiten Stufe mit Verbindungen der Formel 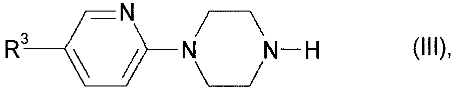 in welcher
- R³: wie zuvor definiert ist,
in Gegenwart von Dehydratisierungsreagenzien.

Die Umsetzung in der ersten Stufe erfolgt im Allgemeinen in inerten Lösungsmitteln, bevorzugt in einem Temperaturbereich von 0°C bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat. Natriumhydroxid ist dabei bevorzugt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemische von Lösungsmitteln mit Wasser. Als Lösungsmittel ist ein Gemisch aus Ethanol und Wasser bevorzugt.

Die Umsetzung in der zweiten Stufe erfolgt im Allgemeinen in inerten Lösungsmitteln, gegebenenfalls in Gegenwart einer Base, bevorzugt in einem Temperaturbereich von -70°C bis 40°C bei Normaldruck.

Als Dehydratisierungsreagenzien eignen sich hierbei beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylaminoisopropyl)-*N'*-ethylcarbodiimid-Hydrochlorid (EDC), N-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)phosphorylchlorid oder Benzotriazolyloxy-tri(dimethylamino)phosphoniumhexafluorophosphat, oder O-(Benzotriazol-1-yl)-*N*,*N*,*N*',*N*'-tetramethyluroniumhexafluorophosphat (HBTU), 2-(2-Oxo1-(2H)-pyridyl)-1,1,3,3-tetramethyluroniumtetrafluoroborat (TPTU) oder O-(7-Azabenzotriazol-1-yl)-*N*,*N*,*N'*,*N'*-tetramethyluroniumhexafluorophosphat (HATU), oder 1-Hydroxybenztriazol (HOBt), oder Benzotriazol-1-yloxytris(dimethylamino)phosphoniumhexafluorophosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin oder DBU, DBN oder Pyridin, bevorzugt ist N-Methylmorpholin.

Vorzugsweise wird die Kondensation mit Propanphosphonsäureanhydrid (T3P) in Gegenwart von *N*-Methylmorpholin (NMM) durchgeführt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylformamid.

Die Verbindungen der Formel (II) sind bekannt oder können hergestellt werden, indem Verbindungen der Formel (IV) 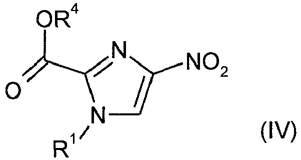 in welcher
R¹ und R⁴ wie zuvor definiert sind,
in der ersten Stufe mit einem Reduktionsmittel und in der zweiten Stufe in Gegenwart eines Kohlensäurederivates mit Verbindungen der Formel

H₂N-R² (V)

in welcher
R² wie zuvor definiert ist,
oder in der zweiten Stufe mit Verbindungen der Formel

OCN-R² (VI),

in welcher
R² wie zuvor definiert ist,
umgesetzt werden.

Die Umsetzung in der ersten Stufe erfolgt dabei im Allgemeinen in inerten Lösungsmitteln, bevorzugt in einem Temperaturbereich von 0°C bis zum Rückfluss der Lösungsmittel bei Normaldruck bis 3 bar.

Reduktionsmittel sind beispielsweise Palladium auf Aktivkohle und Wasserstoff, Ameisensäure/Triethylamin/Palladium auf Aktivkohle, Zink, Zink/Salzsäure, Eisen, Eisen/Salzsäure, Eisen(II)sulfat/Salzsäure, Natriumsulfid, Natriumdisulfid Natriumdithionit, Ammoniumpolysulfid, Natriumborhydrid/Nickelchlorid, Zinndichlorid, Titantrichlorid oder Raney-Nickel und wässrige Hydrazin-Lösung, bevorzugt ist Raney-Nickel und wässrige Hydrazin-Lösung, Palladium auf Aktivkohle und Wasserstoff oder Ameisensäure/Triethylamin/Palladium auf Aktivkohle.

Inerte Lösungsmittel sind beispielsweise Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, im Falle von wassermischbaren Lösungsmitteln auch Gemische derselben mit Wasser. Als Lösungsmittel sind Methanol, Ethanol, iso-Propanol oder im Falle von Raney-Nickel und wässrige Hydrazin-Lösung Tetrahydrofuran bevorzugt.

Die Umsetzung in der zweiten Stufe gemäß der ersten Variante erfolgt im Allgemeinen in inerten Lösungsmitteln, bevorzugt in einem Temperaturbereich von Raumtemperatur bis 40°C bei Normaldruck.

Kohlensäurederivate sind beispielsweise N,N-Carbonyldiimidazol, Phosgen, Diphosgen, Triphosgen, Chlorameisensäurephenylester oder Chlorameisensäure-4-nitrophenylester, bevorzugt ist N,N-Carbonyldiimidazol.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylsulfoxid.

Die Umsetzung in der zweiten Stufe gemäß der zweiten Variante erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt sind Tetrahydrofuran oder Methylenchlorid.

Basen sind beispielsweise Alkalicarbonate wie Cäsiumcarbonat, Natrium- oder Kaliumcarbonat, oder Kalium-*tert*.-butanolat, oder andere Basen wie Natriumhydrid, DBU, Triethylamin oder Diisopropylethylamin, bevorzugt Triethylamin.

Die Verbindungen der Formel (IV) sind bekannt oder können hergestellt werden, indem Verbindungen der Formel 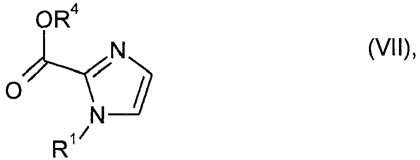 in welcher
R¹ und R⁴ wie zuvor definiert sind
mit rauchender Salpetersäure, konzentrierter Salpetersäure, Nitriersäure oder anderen Mischungsverhältnissen von Schwefelsäure und Salpetersäure, gegebenenfalls in Acetanhydrid als Lösungsmittel, bevorzugt in einem Temperaturbereich von Raumtemperatur bis 60°C bei Normaldruck, umgesetzt werden.

Die Verbindungen der Formeln (III), (IV), (V), (VII) sind bekannt oder lassen sich nach bekannten Verfahren aus den entsprechenden Edukten synthetisieren.

Die Herstellung der zur Herstellung der erfindungsgemäßen Salze verwendeten Heterocyclylamid-substituierten Imidazole ist in dem nachstehenden Syntheseschema exemplarisch näher erläutert. Das Syntheseschema ist hierbei rein beispielhaft und in keiner Weise einschränkend zu verstehen.

### Syntheseschema:

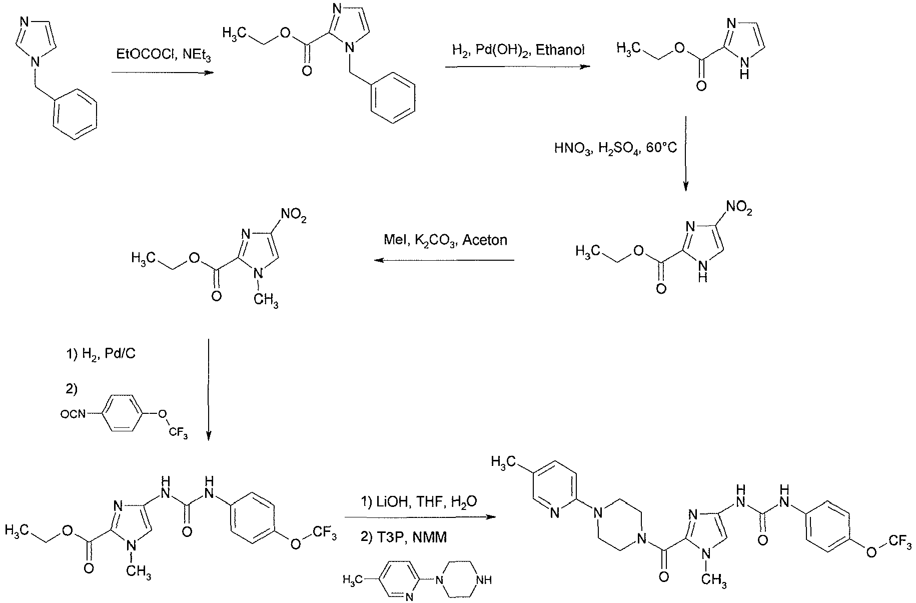

Die erfindungsgemäßen Salze zeigen eine antivirale Wirkung gegenüber Vertretern der Gruppe der Herpes viridae (Herpesviren), vor allem gegenüber Cytomegalieviren (CMV), insbesondere gegenüber dem humanen Cytomegalievirus (HCMV). Sie sind somit geeignet zur Behandlung und/oder Prophylaxe von Krankheiten, vor allem von Infektionen mit Viren, insbesondere den hierin genannten Viren, und den dadurch hervorgerufenen Infektionskrankheiten. Unter einer Virusinfektion wird hierin sowohl eine Infektion mit einem Virus als auch eine durch eine Infektion mit einem Virus hervorgerufene Krankheit verstanden.

Die erfindungsgemäßen Salze können aufgrund ihrer besonderen Eigenschaften zur Herstellung von Arzneimitteln verwendet werden, die zur Prophylaxe und/oder Behandlung von Krankheiten, insbesondere Virusinfektionen, geeignet sind.

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.

Behandlung von HCMV-positiven Krebspatienten mit dem Ziel, HCMVvermittelte Tumorprogression zu verringern (vgl. J. Cinatl, et al., FEMS Microbiology Reviews 2004, 28, 59-77).

Bevorzugt werden die erfindungsgemäßen Salze zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalievirus, geeignet sind.

Die erfindungsgemäßen Salze können aufgrund ihrer pharmakologischen Eigenschaften allein und bei Bedarf auch in Kombination mit anderen Wirkstoffen, insbesondere antiviralen Wirkstoffen wie beispielsweise Valganciclovir, Ganciclovir, Valacyclovir, Acyclovir, Foscarnet, Cidofovir und verwandte Derivate zur Behandlung und/oder Prävention von Virusinfektionen, insbesondere von HCMV-Infektionen, eingesetzt werden.

Weiterer Gegenstand der vorliegenden Offenbarung ist der Einsatz der erfindungsgemäßen Salze in einem Verfahren zur Behandlung und/oder Prophylaxe von Erkrankungen, vorzugsweise von Virusinfektionen, insbesondere von Infektionen mit dem humanen Cytomegalievirus (HCMV) oder einem anderen Vertreter der Gruppe der Herpes viridae.

Weiterer Gegenstand der vorliegenden Offenbarung ist die Verwendung der erfindungsgemäßen Salze zur Behandlung und/oder Prophylaxe von Erkrankungen, insbesondere der zuvor genannten Erkrankungen.

Weiterer Gegenstand der vorliegenden Erfindung sind die erfindungsgemäßen Salze zur Verwendung in einem Verfahren zur Behandlung und/oder Prophylaxe von Erkrankungen, insbesondere der zuvor genannten Erkrankungen.

Weiterer Gegenstand der vorliegenden Offenbarung ist ein Verfahren zur Behandlung und/oder Prophylaxe von Erkrankungen, insbesondere der zuvor genannten Erkrankungen, unter Verwendung einer antiviral wirksamen Menge der erfindungsgemäβen Salze.

Die erfindungsgemäßen Salze können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Salze in geeigneten Applikationsformen verabreicht werden.

Für die orale Applikation eignen sich nach dem Stand der Technik funktionierende schnell und/oder modifiziert die erfindungsgemäßen Salze abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Salze können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglykole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Bevorzugt im Rahmen der Erfindung ist ein Arzneimittel, dass 5 bis 12,5 mg/ml eines erfindungsgemäßen Salzes, 50 bis 150 mg/ml Hydroxypropyl-β-cyclodextrin, 0.5 bis 2.0 mg/ml Natriumacetat sowie Wasser und ggf. weitere pharmazeutisch unbedenkliche Hilfsstoffe aufweist.

Weiterer Gegenstand der vorliegenden Erfindung sind Arzneimittel, die mindestens ein erfindungsgemäßes Salz, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen, bezogen auf den reinen Wirkstoff, von etwa 0,001 bis 10 mg/kg, vorzugsweise etwa 0,01 bis 5 mg/kg Körpergewicht zur Erzielung wirksamer Ergebnisse zu verabreichen. Bei oraler Applikation beträgt die Dosierung üblicherweise etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Erfindung wird nun nachstehend anhand von Beispielen sowie in Bezug auf die beiliegende Zeichnung näher erläutert. Es zeigt:
- Fig. 1:: ein Pulver-XRD-Diffraktogramm des Salzes von Beispiel 1.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Beispiele

**Verwendete Abkürzungen:**
- Bsp.: Beispiel
- DC: Dünnschichtchromatographie
- DMF: *N,N*-Dimethylformamid
- DMSO: Dimethylsulfoxid
- d. Th.: der Theorie
- El: Elektronenstoß-Ionisation (bei MS)
- ESI: Elektrospray-Ionisation (bei MS)
- h: Stunde
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- RP-HPLC: Reverse Phase HPLC
- RT: Raumtemperatur
- Rₜ: Retentionszeit (bei HPLC)
- TBTU: *O*-(Benzotriazol-1-yl)-*N*,*N*,*N,N*'-tetramethyluronium-tetrafluoroborat
- THF: Tetrahydrofuran

### HPLC- und LC-MS-Methoden:

**Methode** 1 **(LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 I Wasser + 0.5 ml 50%ige Ameisensäure, Eluent B: 1 I Acetonitril + 0.5 ml 50%ige Ameisensäure; Gradient: 0.0 min 90%A → 2.5 min 30%A →3.0 min 5%A →4.5 min 5%A; Fluss: 0.0 min 1 ml/min, 2.5 min/3.0 min/4.5 min 2 ml/min; Ofen: 50°C; UV-Detektion: 208- 400 nm.

**Methode 2 (LC-MS):** Instrument: Micromass Platform LCZ mit HPLC Agilent Serie 1100; Säule: Phenomenex Synergi 2µ Hydro-RP Mercury 20 mm x 4 mm; Eluent A: 1 I Wasser + 0.5 ml 50%ige Ameisensäure, Eluent B: 1 I Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 3 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 I Wasser + 0.5 ml 50%ige Ameisensäure, Eluent B: 1 I Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (LC-MS):** Gerätetyp MS: Micromass ZQ; Gerätetyp HPLC: HP 1100 Series; UV DAD; Säule: Phenomenex Synergi 2µHydro-RP Mercury 20 mm x 4 mm; Eluent A: 1 I Wasser + 0.5 ml 50%ige Ameisensäure, Eluent B: 1 I Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 5 (analytische HPLC):** Säule: Kromasil 100 RP-18, 60 mm x 2.1 mm, 3.5 µm; Eluent A: Wasser + 0.5% Perchlorsäure (70%ig), Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Säulentemperatur: 30°C; Detektion: UV 210 nm.

### Ausgangsverbindungen

### Beispiel 1A

### 1-(Cyclopropylmethyl)-4-[({[4-(trifluormethoxy)phenyl]amino}carbonyl)amino]-1H-imidazol-2-carbonsäure

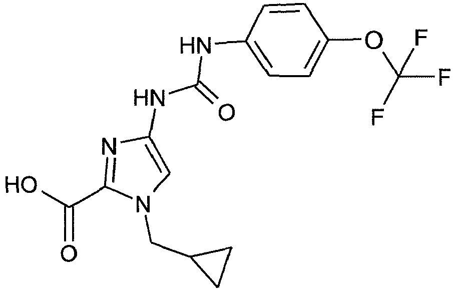

### Stufe 1

### 1-(Cyclopropylmethyl)-4-nitro-1H-imidazol-2-carbonsäureethylester

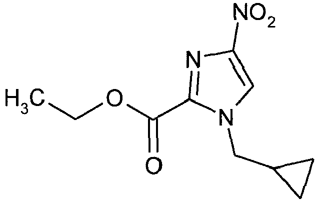

15 g (81 mmol) 4-Nitro-1*H*-imidazol-2-carbonsäureethylester werden unter Argon zusammen mit 13.13 g (97.2 mmol) Cyclopropylmethylbromid und 22.4 g (162 mmol) Kaliumcarbonat in 165 ml DMF 1 h bei 80°C gerührt. Nach Abkühlen wird die Reaktionsmischung mit Wasser verdünnt und viermal mit Ethylacetat extrahiert. Die vereinigten organischen Phasen werden einmal mit Wasser und dreimal mit gesättigter Natriumchloridlösung gewaschen, mit Magnesiumsulfat getrocknet und im Vakuum eingedampft. Der kristalline Rückstand wird für die nächste Reaktion direkt weiterverwendet. Ausbeute: 17.59 g (70 % d. Th.)
LC-MS (Methode 1): Rₜ = 2.02 min.
MS (ESI⁺): m/z = 240 [M+H]⁺
¹H-NMR (300MHz, DMSO-d₆): δ= 8.2 (s, 1H), 4.4 (q, 2H), 4.3 (d, 2H), 1.4 (m, 4H), 0.55 (q, 2H), 0.45 (q, 2H) ppm.

### Stufe 2

### 4-Amino-1-(cyclopropylmethyl)-1H-imidazol-2-carbonsäureethylester

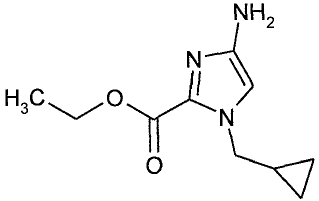

3.89 g (16.26 mmol) 1-(Cyclopropylmethyl)-4-nitro-1*H*-imidazol-2-carbonsäureethylester werden in 50 ml THF gelöst und mit einer Spatelspitze Raney-Nickel versetzt. Die Reaktionsmischung wird in einer Hydrierungsapparatur bei Raumtemperatur mit Wasserstoff hydriert. Der Katalysator wird abfiltriert und das Filtrat im Vakuum eingedampft. Der Eindampfrückstand wird für die nächste Reaktion direkt weiterverwendet.
Ausbeute: 3.46 g (100 % d. Th.)
LC-MS (Methode 2): Rₜ = 1.21 min.
MS (ESI⁺): m/z = 210 [M+H]⁺
¹H-NMR (300MHz, DMSO-d₆): δ = 6.55 (s, 1H), 4.55 (s, 2H), 4.2 (q, 2H), 4.1 (d, 2H), 1.25 (tr, 3H), 1.2 (m, 1H), 0.5 (q, 2H), 0.3 (q, 2H) ppm.

### Stufe 3

### 4-[({[4-(Trifluormethoxy)phenyl]amino}carbonyl)amino]-1-(cyclopropylmethyl)-1H-imidazol-2-carbonsäureethylester

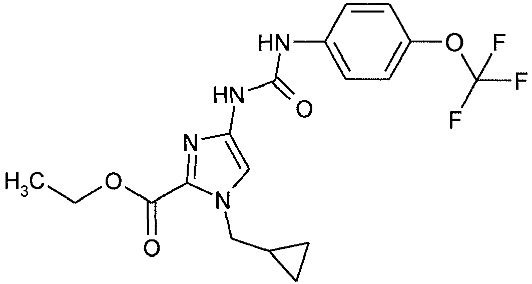

7.49 g (35.8 mmol) 4-Amino-1-(cyclopropylmethyl)-1*H*-imidazol-2-carbon-säureethylester werden in 18 ml THF unter Argon mit 6 g (35.8 mmol) 4-(Trifluormethoxy)phenylisocyanat versetzt und 4 h bei Raumtemperatur gerührt. Die Reaktionsmischung wird im Vakuum eingeengt und das dabei auskristallisierende Produkt in 40 ml Ethylacetat verrührt und abgesaugt.
Ausbeute: 11.1 g (82% d.Th.)
LC-MS (Methode 1): Rₜ = 2.66 min.
MS (ESI⁺): m/z = 376 [M+H]⁺
¹H-NMR (300MHz, DMSO-d₆): δ = 9.45 (s, 1H), 8.0 (d, 1H), 7.35 (s, 1H), 7.3 (d, 1H), 7.2 (dd, 1H), 4.3 (q, 2H), 4.25 (d, 2H), 2.25 (s, 3H), 1.3 (tr, 3H), 1.25 (m, 1H), 0.55 (q, 2H), 0.35 (q, 2H) ppm.

### Stufe 4

### 4-[({[4-(Trifluormethoxy)phenyl]amino}carbonyl)amino]-1-(cyclopropylmethyl)-1H-imidazol-2-carbonsäure

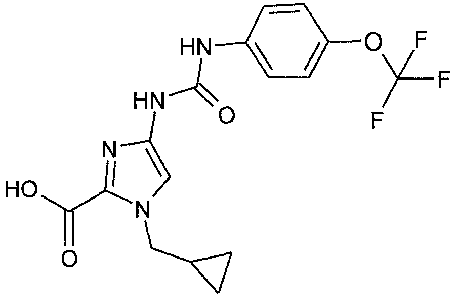

10.6 g (28.1 mmol) 4-[({[4-(Trifluormethoxy)phenyl]amino}carbonyl)amino]-1-(cyclopropylmethyl)-1*H*-imidazol-2-carbonsäureethylester werden in 158 ml Ethanol suspendiert. Unter Eiskühlung werden 16.4 ml Wasser und 6 ml (112 mmol) 50%ige wässrige Natronlauge-Lösung zugegeben. Die Reaktionsmischung wird 1 h bei Raumtemperatur gerührt und dann im Vakuum eingeengt. Der Rückstand wird in 100 ml Isopropanol aufgenommen und unter Eiskühlung mit 100 ml 1N Salzsäure versetzt. Die Kristalle werden abgesaugt und im Vakuum bei 40°C getrocknet.
Ausbeute: 9.85 g (100 % d. Th.)
LC-MS (Methode 3): Rₜ = 1.74 min.
MS (ESI⁺): m/z = 349 [M+H]⁺
¹H-NMR (400MHz, DMSO-d₆): δ = 9.4 (s, 1H), 8.0 (d, 1H), 7.3 (s, 1H), 7.25 (d, 1H), 7.2 (dd, 1H), 4.25 (d, 2H), 2.25 (s, 3H), 1.25 (m, 1H), 0.55 (q, 2H), 0.35 (q, 2H) ppm.

### Beispiel 2A

### 1-Butyl-4-[({[4-(trifluormethoxy)phenyl]amino}carbonyl)amino]-1H-imidazol-2-carbonsäure

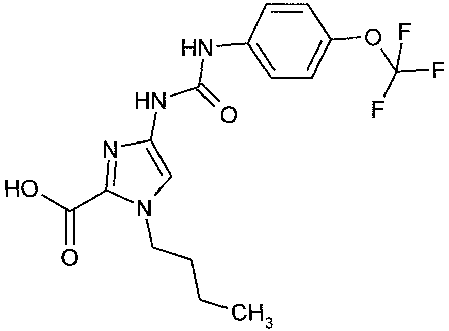

Die Herstellung erfolgt analog zu Beispiel 1A.
Ausbeute: 2.05 g (96 % d. Th.)
LC-MS (Methode 3): Rₜ = 1.96 min.
MS (ESI⁺): m/z = 387 [M+H]⁺
¹H-NMR (300MHz, DMSO-d₆): δ = 9.0 (s, 1H), 8.9 (s, 1H), 7.55 (d, 2H), 7.3 (s, 1H), 7.25 (d, 1H), 4.35 (tr, 2H), 1.7 (quintett, 2H), 1.25 (sextett, 2H), 0.9 (tr, 3H) ppm.

### Beispiel 3A

### 1-Methyl-4-[({[4-(trifluormethoxy)phenyl]amino}carbonyl)amino]-1H-imidazol-2-carbonsäure-ethylester

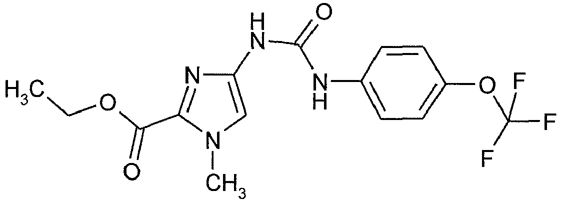

1.22 g (3.61 mmol) 4-Amino-1-methyl-1*H*-imidazol-2-carbonsäureethylester (Synthese analog Beispiel 1A Stufe 3 oder auch gemäß Tetrahedron Lett. 2003, 44, 1607 und dort zitierter Literatur) werden in 50 ml THF unter Argon mit 1.46 g (7.21 mmol) 4-(Trifluormethoxy)phenylisocyanat versetzt und über Nacht bei Raumtemperatur gerührt. Die Reaktionsmischung wird filtriert, das Filtrat im Vakuum eingeengt und chromatographisch gereinigt.
Ausbeute: 860 mg (62 % d. Th.)
LC-MS (Methode 4): Rₜ = 2.41 min.
MS (ESI+): m/z = 373 [M+H]+
¹H-NMR (300MHz, DMSO-d₆): δ = 8.98 (bs, 2H), 7.55 (m, 2H), 7.36 (s, 1H), 7.29 (m, 2H), 4.28 (q, 2H), 3.91 (s, 3H), 1.30 (t, 3H).

### Beispiel 4A

### 1-Methyl-4-[({[4-(trifluormethoxy)phenyl]amino}carbonyl)amino]-1H-imidazol-2-carbonsäure

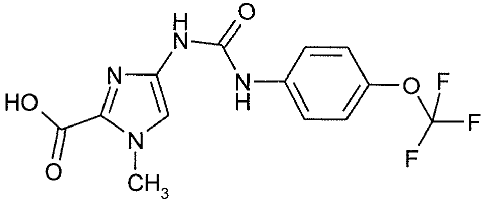

835 mg (2.13 mmol) 1-Methyl-4-[({[4-(trifluormethoxy)phenyl]amino}carbonyl)amino]-1*H*-imidazol-2-carbonsäureethylester (Beispiel 3A) werden in 5 ml Ethanol und 12 ml Tetrahydrofuran suspendiert. Unter Eiskühlung werden 2 ml (25 mmol) 50%ige wässrige Natronlauge-Lösung zugegeben. Die Reaktionsmischung wird über Nacht bei Raumtemperatur gerührt und dann unter Eiskühlung mit 1 N Salzsäure sauer gestellt. Die Lösung wird mit Dichlormethan extrahiert. Die organische Phase wird im Vakuum eingeengt. Der Rückstand wird durch präparative HPLC gereinigt.
Ausbeute: 346 mg (44 % d. Th.).
LC-MS (Methode 3): Rₜ = 1.62 min.
MS (ESI⁺): m/z = 345 [M+H]⁺
¹H-NMR (400MHz, DMSO-d₆): δ = 9.33 (bs, 1 H), 8.98 (bs, 1 H), 7.55 (m, 2H), 7.30 (s, 1 H), 7.28 (m, 2H), 3.90 (s, 3H).

### Beispiel 5A

### 1-Ethyl-4-[({[4-(trifluormethoxy)phenyl]amino}carbonyl)amino]-1H-imidazol-2-carbonsäure

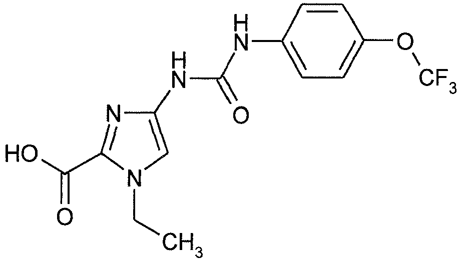

Die Herstellung erfolgt analog zu Beispiel 4A.
Ausbeute: 425 mg (91 % d. Th.).
LC-MS (Methode 4): Rₜ = 1.94 min.
MS (ESI⁺): m/z = 359 [M+H]⁺
¹H-NMR (300MHz, DMSO-d₆): δ = 10.3 (bs, 1H), 7.67 (m, 2H), 7.24 (s, 1H), 7.20 (m, 2H), 4.45 (q, 2H), 1.33 (t, 3H).

### Beispiel 6A

### 4-[({[4-(Difluormethoxy)phenyl]amino}carbonyl)amino]-1-methyl-1H-imidazol-2-carbonsäure

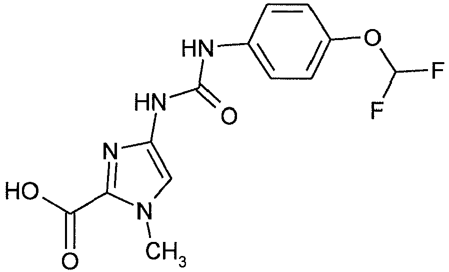

Die Herstellung erfolgt analog zu Beispiel 4A.
Ausbeute: 964 mg (81 % d. Th.).
HPLC (Methode 5): Rₜ = 3.57 min.
MS (ESI⁺): m/z = 327 [M+H]⁺
¹H-NMR (400MHz, CDCl₃): δ = 8.9 (s, 1 H), 8.8 (s, 1H), 7.5 (d, 2H), 7.3 (s, 2H), 7.1 (t, 1H), 7.09 (d, 2H), 3.9 (s, 3H).

### Beispiel 7A

### 1-(5-Methylpyridin-2-yl)piperazin

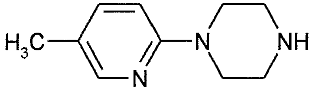

### Stufe 1

### 1-(tert.-Butyloxycarbonyl)-4-(5-methylpyridin-2-yl)piperazin

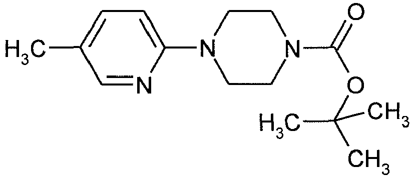

Unter einer Argonatmosphäre werden 2.50 g (19.6 mmol) 2-Methyl-5-chlorpyridin und 4.38 g (23.5 mmol) N-(*tert*.-Butyloxycarbonyl)-piperazin in 50 ml absolutem Toluol gelöst. Anschließend gibt man 2.26 g (23.5 mmol) Natrium-*tert*.-butylat, 0.37 g (0.59 mmol) BINAP und 0.36 g (0.39 mmol) Tris(dibenzylidenaceton)dipalladium hinzu und erhitzt 12 h auf 70°C. Nach dem Abkühlen wird das Reaktionsgemisch mit Diethylether versetzt, dreimal mit gesättigter Natriumchloridlösung gewaschen, über Natriumsulfat getrocknet und im Vakuum vom Lösungsmittel befreit. Der Rückstand wird flashchromatographisch (Cyclohexan/ Ethylacetat 9:1) gereinigt.

Alternativ kann die Kupplungsreaktion auch unter Verwendung von Palladium-(II)-acetat als Katalysator durchgeführt werden.
Ausbeute: 5.27 g (97 % d. Th.).
LC-MS (Methode 3): Rₜ = 1.26 min.
MS (ESI⁺): m/z = 278 [M+H]⁺
¹H-NMR (300MHz, CDCl₃): δ = 8.02 (d, 1H), 7.34 (dd, 1H), 6.59 (d, 1H), 3.55 (m, 4H), 3.45 (m, 4H), 2.21 (s, 3H), 1.49 (s, 9H).

### Stufe 2

### 1-(5-Methylpyridin-2-yl)piperazin

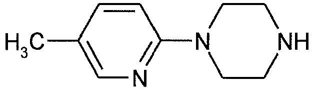

3.47 g (12.5 mmol) 1-(*tert*.-Butyloxycarbonyl)-4-(5-methylpyridin-2-yl)piperazin werden in 10 ml Dioxan gelöst und mit 31 ml (125 mmol) Chlorwasserstoff in Dioxan (4 Molar) versetzt. Man lässt 2 h bei RT rühren. Anschließend wird eingeengt, der Rückstand mit 1 M Natronlauge alkalisiert und mehrmals mit Dichlormethan extrahiert. Die vereinigten organischen Phasen werden über Natriumsulfat getrocknet, eingeengt und im Vakuum getrocknet.

Alternativ kann die Verbindung von Beispiel 7A auch in Form des Hydrochlorid-Salzes isoliert werden.
Ausbeute: 2.18 g (98 % d. Th.).
LC-MS (Methode 4): Rₜ = 0.38 min.
MS(ESI⁺): m/z = 177[M+H]⁺
¹H-NMR (300MHz, CDCl₃): δ = 8.02 (d, 1H), 7.32 (dd, 1H), 6.59 (d, 1H), 3.45 (m, 4H), 3.00 (m, 4H), 2.20 (s, 3H).

### Beispiel 8A

### N-{1-Methyl-2-[(4-pyridin-2-yl-piperazin-1-yl)carbonyl]-1H-imidazol-4-yl}-N'-[4-(trifluormethoxy)phenyl]harnstoff

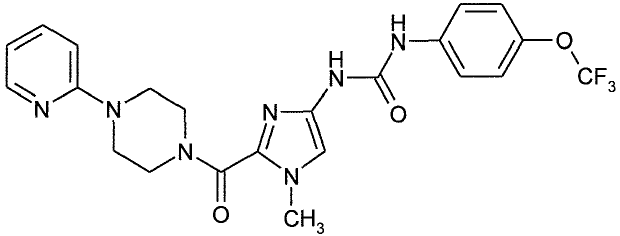

1.50 g (4.36 mmol) der Verbindung aus Beispiel 4A werden in 30 ml DMF gelöst und mit 1.82 g (5.66 mmol) O-(Benzotriazol-1-yl)-N,N,N`,N`-tetramethyluroniumtetrafluoroborat (TBTU) und 266 mg (2.18 mmol) 4-Dimethylaminopyridin versetzt. Nach Zugabe von 925 mg (5.66 mmol) 1-(Pyridin-2-yl)-piperazin lässt man 4 h bei RT rühren. Das Reaktionsgemisch wird durch RP-HPLC gereinigt.
Ausbeute: 1.79 g (83 % d. Th.).
LC-MS (Methode 1): Rₜ= 1.83 min.
MS (ESI⁺): m/z = 490 [M+H]⁺
¹H-NMR (400MHz, DMSO-d₆): δ = 8.89 (bs, 2H), 8.12 (d, 1H), 7.55 (m, 3H), 7.29 (m, 2H), 7.20 (s, 1H), 6.88 (d, 1H), 6.68 (dd, 1H), 4.02 (bs, 2H), 3.77 (s, 3H), 3.71 (bs, 2H), 3.58 (bs, 4H).

### Beispiel 9A

### N-(1-Methyl-2-{[4-(5-methylpyridin-2-yl)piperazin-1-yl]carbonyl)-1H-imidazol-4-yl)-N'-[4-(trifluormethoxy)phenyl]harnstoff

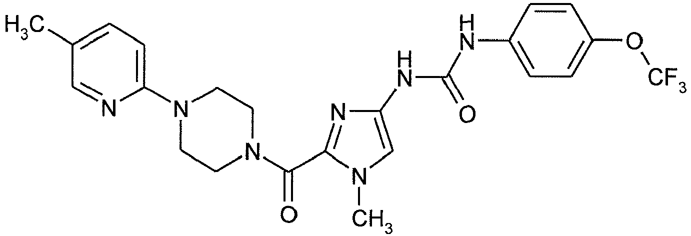

Zu einer Lösung von 9.0 g (26.14 mmol) der Verbindung aus Beispiel 4A in 110 ml Essigsäureethylester werden 5.6 g (26.14 mmol) der Verbindung aus Beispiel 7A und 13.22 g (130.7 mmol) N-Methylmorpholin zugegeben und die Reaktionsmischung wird auf 0°C abgekühlt. Zu der Reaktionslösung werden über 90 min 16.63 g (52.26 mmol) Propanphosphonsäureanhydrid (T3P) zugegeben und die resultierende Suspension wird für weitere 10 min bei dieser Temperatur gerührt. Die Reaktionsmischung wird dann über 60 min auf 20°C erwärmt und bei dieser Temperatur über Nacht gerührt. Unreagiertes T3P wird durch Zugabe von 45 ml Wasser gequencht und die Reaktionsmischung wird für weitere 10 min gerührt. Danach werden die Phasen getrennt und die organische Phase wird mehrmals mit Wasser gewaschen (3 x 45 ml), das auf pH 5 eingestellt ist. Die vereinigten wässrigen Phasen werden noch einmal mit Essigsäureethylester gewaschen und die vereinigten organischen Phasen werden zweimal mit 45 ml wässriger Natriumhydrogencarbonatlösung gewaschen, über Natriumsulfat getrocknet und eingeengt. Das erhaltene Rohprodukt wird aus Ethanol umkristallisiert, wonach das Endprodukt als schwach gelber Feststoff erhalten wird.
Ausbeute: 8.42g (64 % d. Th.).
LC-MS (Methode 4): Rₜ = 2.01 min.
MS (ESI⁺): m/z = 504 [M+H]⁺
¹H-NMR (300MHz, DMSO-d₆): δ = 8.92 (bs, 2H), 7.99 (d, 1H), 7.54 (m, 2H), 7.42 (dd, 1H), 7.28 (m, 2H), 7.20 (s, 1H), 6.80 (d, 1H), 4.00 (bs, 2H), 3.77 (s, 3H), 3.72 (bs, 2H), 3.51 (bs, 4H), 2.16 (s, 3H).

### Beispiel 10A

### N-(2-{[4-(5-Chlorpyridin-2-yl)piperazin-1-yl]carbonyl}-1-ethyl-1H-imidazol-4-yl)-N'-[4-(trifluormethoxy)phenyl]harnstoff

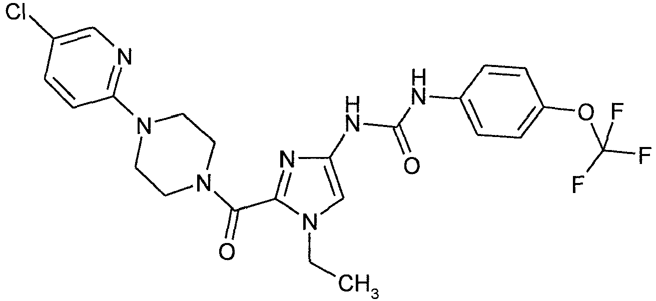

Die Herstellung erfolgt analog zu Beispiel 9A aus Beispiel 5A.
Ausbeute: 55 mg (68 % d. Th.).
LC-MS (Methode 4): Rₜ = 2.76 min.
MS (ESI⁺): m/z = 538 [M+H]⁺
¹H-NMR (300MHz, DMSO-d₆): δ = 8.97 (bs, 1H), 8.92 (bs, 1H), 8.14 (d, 1H), 7.65 (dd, 1H), 7.54 (m, 2H), 7.28 (m, 2H), 7.24 (s, 1H), 6.92 (d, 1H), 4.16 (q, 2H), 3.97 (bs, 2H), 3.72 (bs, 2H), 3.59 (bs, 4H), 1.32 (t, 3H).

### Beispiel 11A

### N-(2-{[4-(4-Methoxyphenyl)piperazin-1-yl]carbonyl}-1-methyl-1H-imidazot-4-yl)-N'-[4-(trifluormethoxy)phenyl]harnstoff

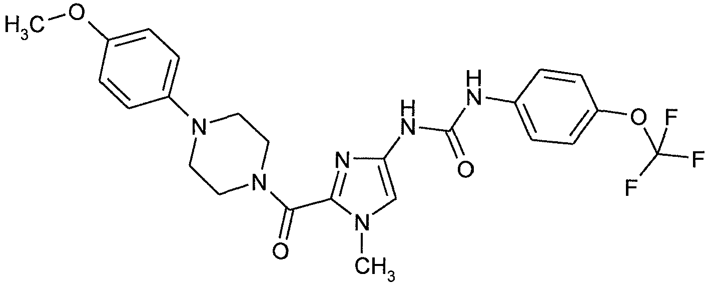

Die Herstellung erfolgt analog zu Beispiel 9A aus Beispiel 4A.
Ausbeute: 35 mg (58 % d. Th.).
LC-MS (Methode 3): Rₜ = 2.24 min.
MS (ESI⁺): m/z = 519 [M+H]⁺
¹H-NMR (400MHz, DMSO-d₆): δ = 8.89 (bs, 2H), 7.53 (m, 2H), 7.28 (m, 2H), 7.19 (s, 1H), 6.92 (m, 2H), 6.84 (m, 2H), 4.05 (bs, 2H), 3.75 (m, 5H), 3.69 (s, 3H), 3.08 (bs, 4H).

### Beispiel 12A

### N-[4-(Difluormethoxy)phenyl]-N'-(1-methyl-2-{[4-(5-methylpyridin-2-yl)piperazin-1-yl]-carbonyl}-1H-imidazol-4-yl)harnstoff

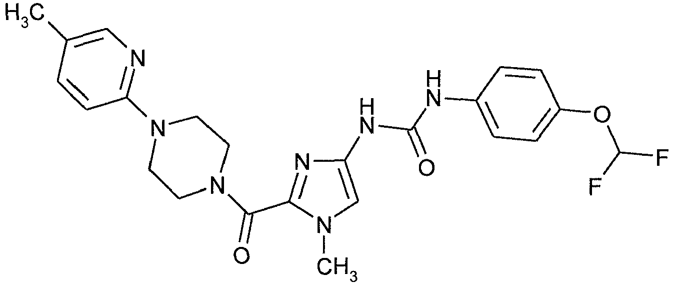

Die Herstellung erfolgt analog zu Beispiel 9A aus Beispiel 6A.
Ausbeute: 17 mg (29 % d. Th.).
LC-MS (Methode 4): Rₜ = 1.70 min.
MS (ESI⁺): m/z = 486 [M+H]⁺
¹H-NMR (400MHz, DMSO-d₆): δ = 8.84 (bs, 1 H), 8.77 (bs, 1 H), 7.98 (d, 1 H), 7.47 (m, 2H), 7.42 (dd, 1H), 7.18 (s, 1H), 7.11 (t, 1H), 7.10 (m, 2H), 6.80 (d, 1H), 4.01 (bs, 2H), 3.77 (s, 3H), 3.71 (bs, 2H), 3.50 (bs, 4H), 2.16 (s, 3H).

Die Beispiele der Tabelle 1 werden analog zu Beispiel 8A hergestellt.

**Tabelle 1**

| **Bsp.-Nr.** | **Struktur** | **Molmasse** | **MS (ESI) [M+H]⁺** | **LC-MS Rₜ [min] (Methode)** | **Ausgangsverbindung** | **Ausbeute (% d. Th.)** |
|---|---|---|---|---|---|---|
| 13A | 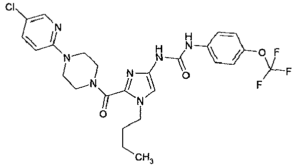 | 565.981 | 566 | 2.94 (4) | Beispiel 2A | 72 |
| 14A | 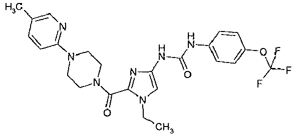 | 517.509 | 518 | 1.94 (4) | Beispiel 5A | 22 |
| 15A | 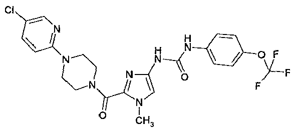 | 523.901 | 524 | 2.67 (4) | Beispiel 4A | 50 |
| 16A | 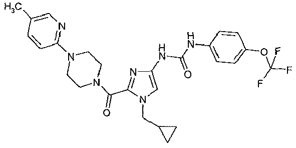 | 543.547 | 544 | 2.11 (4) | Beispiel 1A | 59 |

### Ausführungsbeispiele

### Beispiel 1

### N-(1 -Methyl-2-{[4-(5-methylpyridin-2-yl)piperazin-1-yl]-carbonyl}-1H-imidazol-4-yl)-N'-[4-trifluormethoxyphenyl]harnstoff Dimesylat

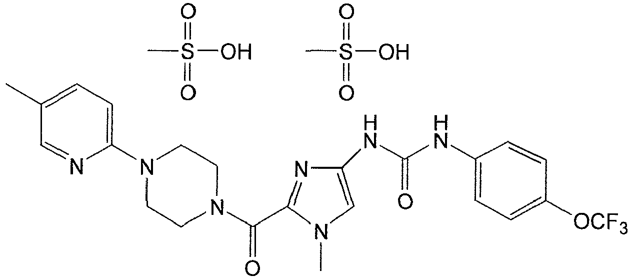

Alle Arbeiten werden unter einer Stickstoff-Schutzgasatmosphäre durchgeführt. In einem Reaktionskessel werden 3202 g der Verbindung von Beispiel 9A (6,36 mol, 1 eq.) mit einer Mischung aus 15 I THF und 1 I Wasser vermischt. Die resultierende Suspension wird langsam auf 60°C erwärmt und dann 30 min bei dieser Temperatur gerührt. Zu der gebildeten gelblichen Lösung werden über 30 min 1252 g Methansulfonsäure (13,03 mol, 2,05 eq.) zugegeben und danach wird mit N-(1-Methyl-2-{[4-(5-methyl-pyridin-2-yl)piperazin-1-yl]-carbonyl}-1*H*-imidazol-4-yl)-N'-[4-trifluormethoxyphenyl]harnstoff Dimesylat angeimpft. Zu der entstehenden Suspension von kristallinem N-(1-Methyl-2-{[4-(5-methylpyridin-2-yl)piperazin-1-yl]-carbonyl}-1*H*-imidazol-4-yl)-N'-[4-trifluormethoxyphenyl]harnstoff Dimesylat werden über 2 Stunden weitere 30 I THF zugegeben. Die Suspension wird langsam auf 20°C abgekühlt und dann für weitere 12 h bei dieser Temperatur gerührt. Die gebildeten Kristalle werden durch Vakuumfiltration gesammelt und der Reaktor wird mit THF und anschließend n-Heptan nachgespült, wobei diese organischen Phasen dann zum Waschen der Kristalle verwendet werden. Zum Abschluss werden die Kristalle unter Vakuum und einem Stickstoffstrom auf dem Filter getrocknet. Es werden 4262g (Ausbeute: 96,4 %, Reinheit > 99 %) des gewünschten Dimesylat-Salzes erhalten.
¹H-NMR (400MHz, DMSO-d₆): δ = 9.07 (s, 1H), 8.98 (s, 1H), 7.99 (s, 1H), 7.92 (d, 1H), 7.56 (d, 2H), 7.41 (d, 1H), 7.33-7.24 (m, 3H), 4.18 (s, br., 2H), 3.92-3.69 (m, 9H), 2.43-2.39 (s, 6H), 2.25 (s, 3H).

Unter Verwendung eines Rigaku MiniFlex Pulver-XRD-Spektrometers wurde das in Fig. 1 dargestellte Röntgendiffraktogramm aufgenommen.

Die Verbindungen der Beispiele 8A sowie 10A bis 15A können in analoger Weise in die Dimesylat-Salze überführt werden.

### Löslichkeitsstudien

20 mg der Verbindung von Beispiel 1 sowie zum Vergleich die Citrat-, Maleat-, Sulfat- und Tartratsalze der Verbindung von Beispiel 9A sowie Chloridsalze der Verbindung von Beispiel 9A, die mit 1, 2 und 4 eq. Chlorwasserstoffsäure hergestellt wurden, sowie die freie Base werden in HPLC-Gläschen eingewogen, die mit einem Magnetrührer ausgestattet waren. Es werden je 1 ml H₂O zugegeben und die HPLC-Gläschen werden verschlossen. Die entstehenden Suspensionen werden über Nacht bei 25°C gerührt. Zur Abschätzung der Menge an gelöster Substanz werden die Suspensionen über Pipettenmikrofilter filtriert und die erhaltenen Filtrate werden 1:4 verdünnt und mittels HPLC analysiert. Die HPLC-Analyse erfolgte auf einer Dionex Luna RP18 (100A)-Säule mit den folgenden Abmessungen: 5µm 50x4,6 mm unter Verwendung einer isokratischen Mischung aus Acetonitril und H₂O + 0,1 % TFA im Verhältnis 3:7.

Als Ergebnis der Löslichkeitsmessungen wurden die nachstehend Tabelle 2 dargestellten Werte ermittelt.

**Tabelle 2**

| **Verbindung** | **Löslichkeit [mg/ml]** |
|---|---|
| Bsp. 9A, freie Base | 0,004 |
| Bsp. 9A Citrat | 0,42 |
| Bsp. 9A Maleat | 0,12 |
| Bsp. 9A Sulfat | 0,18 |
| Bsp. 9A Tartrat | 0,72 |
| Bsp. 9A Chlorid 1 eq. | 0,19 |
| Bsp. 9A Chlorid 2 eq. | 0,07 |
| Bsp. 9A Chlorid 4 eq. | 0,11 |
| Bsp.1 | >9,70 |

Diese Werte zeigen deutlich die überlegene Löslichkeit des Salzes von Beispiel 1 in wässrigem Medium gegenüber anderen Salzen der Verbindung von Beispiel 9A.

### Löslichkeit unter simulierten Bedingungen im menschlichen Magen

Zur Bestimmung der Löslichkeit unter simulierten Bedingungen im menschlichen Magen werden Aufschlämmungen des Salzes von Beispiel 1 sowie der Citrat- und Tartratsalze von Beispiel 9A und der entsprechenden freien Base in wässriger Natriumchloridlösung (0,2 Gew.-%), die mit Chlorwasserstoffsäure auf einen pH von 1,2 eingestellt wurde, für 5 Stunden gerührt. Die Proben wurden danach wie oben beschrieben behandelt und die Menge an freier Base in Lösung wird mittels HPLC ermittelt. Die entsprechenden Werte für die Löslichkeit unter simulierten Bedingungen des menschlichen Magens sind nachstehend in Tabelle 3 dargestellt.

**Tabelle 3**

| Verbindung | Menge an gelöster freier Base [mg/ml] |
|---|---|
| Bsp. 9A Citrat | 0,068 |
| Bsp. 9A Tartrat | 0,047 |
| Bsp. 9A, freie Base | 0,065 |
| Bsp. 1 | 0,103 |

Diese Tabelle zeigt klar die deutlich bessere Löslichkeit der erfindungsgemäßen Salze unter simulierten Bedingungen im menschlichen Magen. Es ist hierbei anzumerken, dass nach Stehenlassen der Lösung für längere Zeiträume das erneute Auftreten einer Suspension beobachtet werden konnte. Es steht zu vermuten, dass dieses aus der Bildung von schlechter löslichen Chloridsalzen von Beispiel 9A herrührt. Diese beobachtete Bildung von unlöslichen Chloridsalzen wurde aber als nicht schwerwiegend eingeschätzt, da diese-vor allem bei Verwendung von Beispiel 1 - mit Verzögerung eintritt und somit zunächst eine metastabile übersättigte Lösung vorliegt. Dies ist ein weiteres Indiz für die Vorteile, die mit der Verwendung der erfindungsgemäßen Salze zur Herstellung von Medikamenten erreicht werden können.

### Hygroskopie

Durch Lagern des Salzes von Beispiel 1 in reiner Form bei etwa 46 % relativer Luftfeuchtigkeit und 24°C wurde die Hygroskopie der erfindungsgemäßen Salze vermessen. Das Salz von Beispiel 1 zeigte dabei nach einer Lagerzeit von ca. 2 Tagen eine Gewichtszunahme von weniger als 0,11 %, was eine für die Verwendung in Medikamenten annehmbare Hygroskopie darstellt.

### B. Bewertung der physiologischen Wirksamkeit

Die *in-vitro-Wirkung* der erfindungsgemäßen Verbindungen auf die Replikation des HCMV (humanes Cytomegalovirus) kann in folgendem antiviralen Assay gezeigt werden:

### HCMV Fluoreszenzreduktionstest

Die Testverbindungen werden als 50 millimolare (mM) Lösungen in Dimethylsulfoxid (DMSO) eingesetzt. Als Referenzverbindungen können z.B. Ganciclovir®, Foscarnet® Cidofovir® oder auch die Verbindung von Beispiel 9A verwendet werden. Einen Tag vor Testansatz werden 1,5 x 10⁴ humane Vorhautfibroblasten (NHDF-Zellen)/well in 200 µl Zellkulturmedium in die Wells B2-G11 von 96-well Platten (schwarz mit durchsichtigem Boden) ausgesät. Die randständigen Wells jeder 96-well Platte werden lediglich mit 200µl Medium befüllt um Randeffekte zu vermeiden. Am Versuchstag wird das Zellkulturmedium der Wells B2-G11 jeder 96-well Platte abgesaugt und durch 100 µl Virussuspension ersetzt (multiplicity of infection (MOI): 0.1-0.2). Bei dem verwendeten Virus handelt es sich um ein rekombinantes HCMV welches eine Expressionskassette für das green fluorescence protein (GFP) in das Virusgenom integriert hat (HCMV AD 169 RV-HG(E. M. Borst, K. Wagner, A. Binz, B. Sodeik, und M. Messerle, 2008, J. Virol. 82:2065-2078.). Nach einer Inkubationszeit von 2h bei 37°C und 5% CO₂ wird das Virus-Inokulat abgesaugt und alle Wells mit Ausnahme der Wells in Spalte 3 werden mit 200 µl Zellkulturmedium befüllt. Spalte 2 wird nicht weiter behandelt und dient als Viruskontrolle. Die Wells in Spalte 3 werden jeweils in Doppelbestimmung mit 300 µlTestsubstanz (verdünnt in Zellkulturmedium) befüllt. Die Konzentration der jeweiligen antiviralen Substanz in Spalte 3 beträgt∼die 27fache Konzentration des jeweils erwarteten EC₅₀ Werts. Die Testsubstanz in Spalte 3 wird in 8 Schritten 1:3 über die 96-well Platte verdünnt indem jeweils 100 µl einer Spalte in die jeweils rechte Spalte transferiert- und dort mit den vorhandenen 200 µl Zellkulturmedium gemischt werden. Auf diese Weise werden drei antivirale Substanzen in Doppelbestimmungen getestet. Die Platten werden 7 Tage bei 37°C / 5% CO₂ inkubiert. Im Anschluss werden alle Wells einer Platte 3 x mit PBS (Phosphate Buffered Saline) gewaschen und mit 50 µl PBS befüllt. Danach wird die GFP-Intensität jedes Wells einer 96-well Platte mittels eines Fluoreszenzlesegerätes (FluoBox; Bayer Technology Services GmbH; Filtereinstellungen: GFP, Ex 480 nm, Em 520 nm) bestimmt. Aus den so erhaltenen Messwerten kann der EC₅₀ einer anti-HCMV-Substanz ermittelt werden:

EC₅₀ (GFP-RA) = Substanzkonzentration in µM welche im Vergleich zur unbehandelten Viruskontrolle die GFP-Fluoreszenz in infizierten Zellen um 50% reduziert.

Repräsentative in-*vitro*-Wirkdaten für die erfindungsgemäßen Verbindungen sind in Tabelle 4 wiedergegeben:

**Tabelle 4**

| **Virus-Stamm** | **Beispiel 9A EC₅₀ [µM]** | **Beispiel 1 EC₅₀ [µM]** | **Ganciclovir EC₅₀ [**µ**M]** |
|---|---|---|---|
| AD169 RV-HG | 0.0015 | 0.0018 | 3.2 |

### Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

5,53 g der Verbindung aus Beispiel 1, 1000 g Wasser für Injektionszwecke, das 10% (w/v) Hydroxypropyl-β-cyclodextrin (Aldrich) enthält, und 985 mg Natriumacetat.

### Herstellung:

Die erfindungsgemäße Verbindung wird unter Rühren in dem Wasser gelöst und der pH-Wert der Lösung mit dem Natriumacetat auf einen pH-Wert von ca. 3,94 eingestellt. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Salz einer Verbindung der Formel 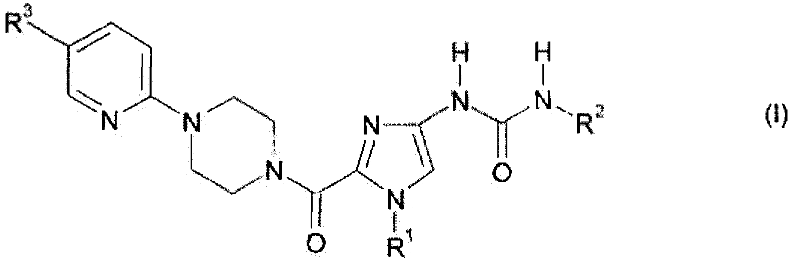 in welcher
R¹ für Methyl, Ethyl, Butyl oder Cyclopropylmethyl steht,
R² für Phenyl steht, wobei Phenyl substituiert ist mit einem Substituenten ausgewählt aus der Gruppe bestehend aus Trifluormethoxy und Difluormethoxy, und
R³ für Wasserstoff, Methyl, Chlor, Methoxy oder Trifluormethyl steht,
mit Methansulfonsäure.

2. Salz nach Anspruch 1, **dadurch gekennzeichnet, dass** es ein Dimesylatsalz ist.

3. Salz nach Anspruch 2 mit der folgenden Formel:

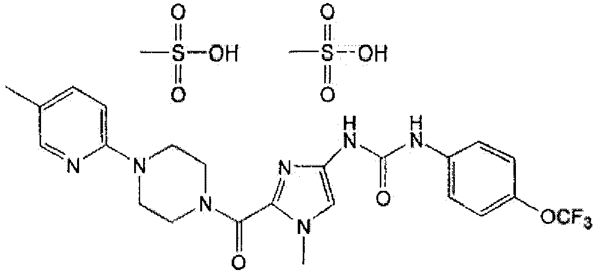

4. Salz nach einem der Ansprüche 1 bis 3, **gekennzeichnet durch** kristallines N-(1-Methyl-2-{[4-(5-methylpyridin-2-yl)piperazin-1-yl]-carbonyl}-1H-imidazol-4-yl)-N'-[4-trifluormethoxyphenyl]harnstoff Dimesylat, weiter **dadurch** gekennzeichnet, dass das Pulver-XRD-Diffraktogramm charakteristische Peaks bei 6.37, 11.77, 12.56, 17.17, 18.81, 20.34, 21.47, 23.04, 35.46 Grad 2Theta zeigt, wenn es mit einem Rigaku MiniFlex Pulver-XRD-Spektrometer aufgenommen wird.

5. Salz nach einem der Ansprüche 1 bis 4, **gekennzeichnet durch** kristallines N-(1-Methyl-2-{[4-(5-methylpyridin-2-yl)piperazin-1-yl]-carbonyl}-1H-imidazol-4-yl)-N'-[4-trifluormethoxyphenyl]harnstoff Dimesylat, weiter **gekennzeichnet durch** ein Pulver-XRD-Diffraktogramm wie in Fig. 1 dargestellt, wenn es mit einem Rigaku MiniFlex Pulver-XRD-Spektrometer aufgenommen wird.

6. Verfahren zur Herstellung eines Salzes der Verbindungen der Formel (I) nach Anspruch 1, das die Umsetzung einer Verbindung der Formel (I) mit Methansulfonsäure aufweist.

7. Salz nach einem der Ansprüche 1 bis 5 zur Verwendung in einem Verfahren zur Behandlung und/oder Prophylaxe von Krankheiten, insbesondere von Virusinfektionen, vorzugsweise von Infektionen mit HCMV oder einem anderen Vertreter der Gruppe der Herpes viridae.

8. Arzneimittel enthaltend ein Salz nach einem der Ansprüche 1 bis 5 in Kombination mit mindestens einem inerten, nichttoxischen, pharmazeutisch geeigneten Hilfsstoff.

9. Arzneimittel nach Anspruch 8, **dadurch gekennzeichnet, dass** es 5 bis 12,5 mg/ml eines Salzes nach einem der Ansprüche 1 bis 5, 50 bis 150 mg/ml Hydroxypropyl-β-cyclodextrin, 0.5 bis 2.0 mg/ml Natriumacetat sowie Wasser und ggf. weitere pharmazeutisch unbedenkliche Hilfsstoffe aufweist.

10. Arzneimittel nach Anspruch 8 oder 9 zur Verwendung in einem Verfahren zur Behandlung und/oder Prophylaxe von Virusinfektionen, vorzugsweise von Infektionen mit HCMV oder einem anderen Vertreter der Gruppe der Herpes viridae.

## Claims

1. A salt of a compound of the formula 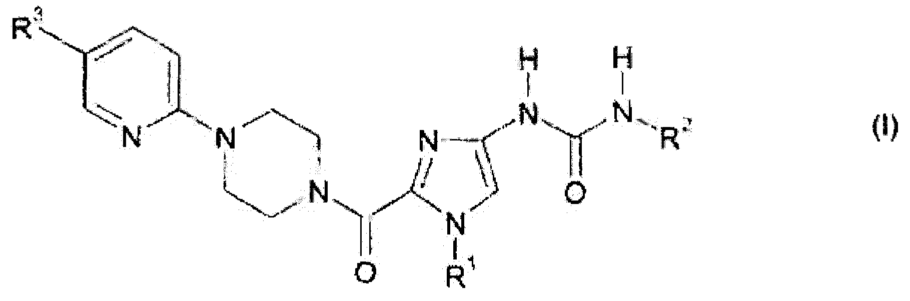 in which
R¹ stands for methyl, ethyl, butyl or cyclopropylmethyl,
R² stands for phenyl, where phenyl is substituted with a substituent selected from the group consisting of trifluoromethoxy and difluoromethoxy, and
R³ stands for hydrogen, methyl, chlorine, methoxy or trifluoromethyl,
with methanesulfonic acid.

2. The salt according to Claim 1, **characterized in that** it is a dimesylate salt.

3. The salt according to Claim 2 with the following formula:

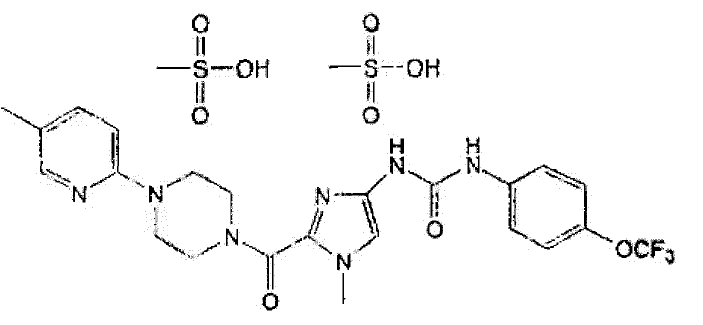

4. The salt according to any of Claims 1 to 3, **characterized by** crystalline N-(1-methyl-2-{[4-(5-methylpyridine-2-yl)piperazine-1-yl]-carbonyl}-1H-imidazole-4-yl)-N'-[4-trifluoromethoxyphenyl]urea dimesylate, further **characterized in that** the X-ray powder diffractogram (XRD) shows characteristic peaks at 6.37,11.77, 12.56,17.17, 18.81, 20.34, 21.47, 23.04, 35.46 degrees 2-theta when recorded with a Rigaku MiniFlex X-ray powder spectrometer (XRD).

5. The salt according to any of Claims 1 to 4, **characterized by** crystalline N-(1-methyl-2-{[4-(5-methylpyridine-2-yl)piperazine-1-yl]-carbonyl}-1H-imidazole-4-yl)-N'-(4-trifluoromelhoxyphenyl]urea dimesylate, further **characterized by** an X-ray powder diffractogram (XRD) as illustrated in Fig. 1, when recorded with a Rigaku MiniFlex X-ray powder spectrometer (XRD).

6. A method for the production of a salt of the compounds of Formula (I) according to Claim 1, which features the reaction of a compound of Formula (I) with methanesulfonic acid.

7. The salt according to any of Claims 1 to 5 for use in a method for treatment and/or prophylaxis of diseases, in particular of virus infections, preferably human cytomegalovirus (HCMV) infections or infections with another representative of the herpes viridae group.

8. A drug containing a salt according to any of Claims 1 to 5 in combination with at least one inert, non-toxic, pharmaceutically suitable excipient.

9. The drug according to Claim 8, **characterized in that** it features 5 to 12.5 mg/mL of a salt according to any of Claims 1 to 5, 50 to 150 mg/mL hydroxypropyl-β-cyclodextrin, 0.5 to 2.0 mg/mL sodium acetate and water and, if applicable, further pharmaceutically acceptable excipients.

10. The drug according to Claim 8 or 9 for use in a method for treatment and/or prophylaxis of virus infections, preferably human cytomegalovirus (HCMV) infections or infections with another representative of the herpes viridae group.

## Revendications

1. Sel d'un composé de la formule 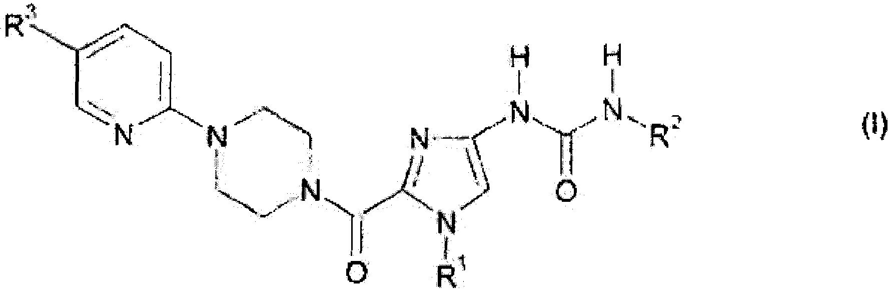 dans laquelle
R¹ représente méthyle, éthyle, butyle ou cyclopropylméthyle;
R² représente phényle, celui-ci étant substitué par un substituant choisi parmi le groupe comprenant trifluorométhoxy et difluorométhoxy; et
R³ représente hydrogène, méthyle, chlore, méthoxy ou trifluorométhyle;
avec l'acide méthanesulfonique.

2. Sel selon la revendication 1, **caractérisé en ce qu'**il s'agit d'un sel de dimésylate.

3. Sel selon la revendication 2 avec la formule suivante :

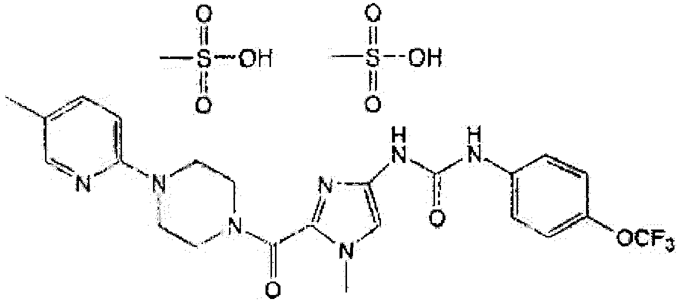

4. Sel selon une quelconque des revendications 1 à 3, **caractérisé par** le dimésylate d'urée N-(1-méthyl-2-{[4-(5-méthylpyridine-2-yl)pipérazine-1-yl]-carbonyle}-1H-imidazole-4-yl)-N'-[4-trifluorométhoxyphényle] cristallin, **caractérisé en outre en ce que** le diffractogramme de poudre par rayons X (XRD) montre des pics caractéristiques à 6,37; 11,77; 12,56; 17,17; 18,81; 20,34; 21,47; 23,04; 35,46 degrés 2 thêta lorsqu'il est mesuré à l'aide d'un spectromètre de poudre par rayons X (XRD) Rigaku Miniflex.

5. Sel selon une quelconque des revendications 1 à 4, **caractérisé par** le dimésylate d'urée N-(1-méthyl-2-{[4-(5-méthylpyridine-2-yl)pipérazine-1-yl]-carbonyle}-1H-imidazole-4-yl)-N'-[4-trifluorométhoxyphenyle] cristallin, **caractérisé en outre par** le diffractogramme de poudre par rayons X (XRD), comme l'illustrédans !a figure 1, lorsqu'il est mesuré à l'aide d'un spectromètre de poudre par rayons X (XRD) Rigaku Miniflex.

6. Méthode de production d'un sel des composés de la formule (I) selon la revendication 1, ladite méthode comprenant la réaction d'un composé de la formule (I) avec l'acide méthanesulfonique.

7. Sel selon l'une quelconque des revendications 1 à 5, destiné à être utilisé dans une méthode de traitement et/ou de prévention de maladies, en particulier les infections virales, de préférence les infections au cytomégalovirus humain (HCMV) ou les infections par un autre membre de la famille des Herpes viridae.

8. Médicament contenant un sel selon l'une quelconque des revendications 1 à 5 en combinaison avec au moins un excipient inerte, non toxique, pharmaceutiquement acceptable.

9. Médicament selon la revendication 8, **caractérisé en ce qu'**il comprend de 5 à 12,5 mg/mL d'un sel selon une quelconque des revendications 1 à 5, de 50 à 150 mg/mL d'hydroxypropyl-β-cyclodextrine, de 0,5 à 2 mg/mL d'acétate de sodium et d'eau et, si nécessaire, d'autres excipients pharmaceutiquement acceptables.

10. Médicament selon la revendication 8 ou 9, destiné à être utilisé dans une méthode de traitement et/ou de prévention d'infections virales, de préférence les infections au cytomégalovirus humain (HCMV) ou les infections par un autre membre de la famille des Herpes viridae.
